# EUROPEAN PATENT APPLICATION

(11) **EP 3 363 746 A1**
(43) Date of publication of application: **22.08.2018**
(21) Application number: 16855431.9
(22) Date of filing: 12.10.2016
(51) Int. Cl.: B65B 55/04, A61L 2/14, A61L 2/16, B65B 55/10

(54) **STERILIZATION SYSTEM**

(30) Priority: 13.10.2015 JP 2015202175
(71) Applicant: Suntory Holdings Limited, Osaka-shi, Osaka 530-8203 (JP)
(72) Inventor: HIGASHIYAMA Kenichi, Soraku-gun Kyoto 619-0284 (JP); TOMINAGA Kenta, Soraku-gun Kyoto 619-0284 (JP); HIRAYAMA Yuji, Soraku-gun Kyoto 619-0284 (JP); YOSHIHARA Kazuki, Soraku-gun Kyoto 619-0284 (JP); IIZUKA Toshiaki, Tokyo 135-8631 (JP); MORIYA Satoshi, Tokyo 135-8631 (JP)
(74) Representative: Stoner, Gerard Patrick
(86) International application number: PCT/JP2016/080262
(87) International publication number: WO 2017/065178

(57) **Abstract**

A sterilizing system (100) includes a sterilizing device (1) configured to discharge sterilizing agent (70) onto a conveyed sterilization-subject article (80) over a span of a predetermined section (D) and a conveying unit (110) for conveying the sterilization-subject article (80). And, a sterilizing mechanism (X) is provided in the span of the predetermined section (D) and configured to be able to sterilize at least one side (81) and the other side (82) of the sterilization-subject article (80).

## Description

### TECHNICAL FIELD

This disclosure relates to a sterilizing system configured to sterilize a sterilization-subject article such as a cap of a container.

### RELATED ART

For instance, in case a device for sterilizing a sterilization-subject article configured to generate plasma and to sterilize the article with a sterilizing agent containing the generated plasma is employed as a sterilizing device, inside this device, a high voltage is applied for the generation of plasma. Then, if water or the like is mixed therein, an extremely dangerous situation will occur. Thus, when such sterilizing device is used, it is safe to effect sterilization with its outlet for discharging the sterilizing agent oriented downwards. Although certain variation in the orientation may be permissible, it is still not possible to effect the sterilization with the outlet oriented upwards. In such case, the sterilization will be effected by discharging the sterilizing agent onto the subject article, which is conveyed continuously by a conveyer, from above the article.

### SUMMARY

### PROBLEM TO BE SOLVED BY INVENTION

However, in the above case, discharging of the sterilizing agent to the article is possible only from one side thereof. At least, it is difficult to cause the agent to reach the other side portion of the article, so sterilization of such portion is difficult. In this way, it was conventionally difficult to sterilize the entire sterilization-subject article in an efficient manner.

Then, there is a need for realizing a sterilizing system capable of sterilizing a sterilization-subject article entirely in an efficient manner.

### SOLUTION

A sterilizing system relating to the present invention comprises:
a conveying unit for conveying a sterilization-subject article;
a sterilizing device configured to discharge sterilizing agent onto the conveyed sterilization-subject article over a span of a predetermined section; and
a sterilizing mechanism provided in the span of the predetermined section and configured to be able to sterilize at least one side and the other side of the sterilization-subject article.

With the above-described arrangement, since one side and the other of the sterilization-subject article can be sterilized, the article can be sterilized from both sides thereof. Further, respecting a portion of the article located laterally of the one side and the other side thereof, as the sterilizing agent is dispersed around the article; such portion is sterilized by the dispersed sterilizing agent. As a result, the entire sterilization-subject article can be sterilized in an efficient manner.

Next, preferred embodiments of the sterilizing system relating to the present invention will be explained. It is understood, however, that the scope of the present invention is not limited by these preferred embodiments described below.

According to one preferred embodiment, the sterilizing mechanism comprises an inverting mechanism configured to at least invert the sterilization-subject article in the span of the predetermined section.

With the above-described arrangement, since the sterilization-subject article is inverted in the span of the predetermined section, sterilization of the article from one side and the other side thereof is made possible. Thus, the entire sterilization-subject article can be sterilized in an efficient manner. Further, the above arrangement does not require any special additional mechanism on the side of the sterilizing device, but allows the sterilization of the sterilization-subject article from both sides thereof only by inverting this article. Therefore, this arrangement can be suitably applied to a case where the sterilizing agent is discharged in one fixed direction only, without changing the discharging direction of the sterilizing agent.

According to one preferred embodiment,
the conveying unit comprises, at least in the span of the predetermined section, a plurality of rod-like members each extending along a conveying direction;
the plurality of rod-like members are spaced from each other and are arranged in a helical configuration along a shape of the sterilization-subject article;
the sterilization-subject article is conveyed within an inner space delimited by the plurality of rod-like members while being guided by these rod-like members; and
the inverting mechanism is configured to helically invert the respective rod-like members all together in the predetermined section for at least a half rotation.

With the above-described arrangement, in the span of the predetermined section, the respective rod-like members are caused to effect at least a half rotation helically, thus, at least the inner space delimited by the plurality of rod-like members and acting as a conveying path for the sterilization-subject article is inverted. Then, while the sterilization-subject article is being conveyed along the inner space, this article is inverted. And, since the respective rod-like members are spaced apart from each other, the sterilizing agent will be applied to the sterilization-subject article via gaps formed between the respective rod-like members. So, the rod-like members present no obstacle to the sterilization. Thus, sterilization of one side and the other side of the sterilization-subject article is made possible, whereby the entire sterilization-subject article can be sterilized in an efficient manner.

According to one preferred embodiment:
the sterilizing device comprises a plasma sterilizing device configured to generate plasma and to sterilize the sterilization-subject article with the obtained plasma;
the plasma sterilizing device receives supply of steam and the plasma sterilizing device is configured to produce reactive oxygen through reaction between the supplied steam and the generated plasma and to discharge the generated plasma and the produced reactive oxygen as the sterilizing agent; and
an outlet of the plasma sterilizing device for discharging the sterilizing agent is oriented perpendicularly downwards.

Reactive oxygen dies out over time, so it will not remain. Moreover, sterilization using reactive oxygen does not require application of such heat which may cause thermal contraction of the sterilization-subject article. Therefore, by using this plasma sterilizing device that discharges a sterilizing agent containing plasma and reactive oxygen, it is possible to avoid residues of the sterilizing agent on the sterilization-subject article and occurrence of excessive thermal contraction/deformation of the sterilization-subject article.

However, with such sterilization using reactive oxygen, as a high voltage is applied for plasma generation inside the device, mixing intrusion of water will be dangerous. Then, in order to prevent intrusion of water into the device, although variation in the orientation may be permissible within a limit, the outlet for discharging the sterilizing agent needs to be oriented downwards. On the other hand, according to the above-described inventive arrangement, since the plasma sterilizing device is used in the sterilizing system having the inverting mechanism, sterilization of one side and the other side of the sterilization-subject article is made possible by the inverting mechanism while avoiding water intrusion into the device by discharging the sterilizing agent downwards. Thus, the entire sterilization-subject article can be sterilized in an efficient manner.

According to one preferred embodiment, the sterilizing mechanism is configured to vary a discharging angle of the sterilizing agent relative to the conveying unit, in the span of the predetermined section.

With the above-described arrangement, by varying the discharging direction of the sterilizing agent, the sterilizing agent can be discharged in various angles relative to the sterilization-subject article, so that the entire article can be sterilized in an efficient manner. If this arrangement is combined with the inverting mechanism, it becomes advantageously possible to discharge the sterilizing agent to the sterilization-subject article over an even wider angular range. Further, the above arrangement can be applied in the case of using the plasma sterilizing device also, since certain variation of the discharging direction is permissible within a limit that ensures reliable prevention of water intrusion to the device.

### BRIEF DESCRIPTION OF THE DRAWINGS

[Fig.1] is a schematic configuration diagram of a plasma sterilizing device for effecting sterilization using reactive oxygen,
[Fig. 2] is a schematic configuration block diagram of the plasma sterilizing device for effecting sterilization using reactive oxygen, and
[Fig. 3] is a schematic explanatory view of a sterilizing system.

### EMBODIMENT

Next, an embodiment of a sterilizing system relating to the present invention will be explained with reference to the accompanying drawings. A sterilizing system 100 according to the instant embodiment is provided for sterilizing caps 80 of containers such as PET bottles. And, the sterilizing system 100 includes a plurality of plasma sterilizing devices 1 configured to generate plasma and to sterilize the caps 80 by the obtained plasma. Each plasma sterilizing device 1 receives a supply of steam and discharges a sterilizing agent containing reactive oxygen (reactive oxidizing species (ROS), e.g. OH radical or singlet oxygen, etc.) produced from a reaction between the supplied steam and the generated plasma, thus sterilizing the caps 80. Then, firstly, there will be explained the device configuration of the plasma sterilizing device 1 (which may be referred to simply as a "sterilizing device" hereinafter) for effecting sterilization using reactive oxygen.

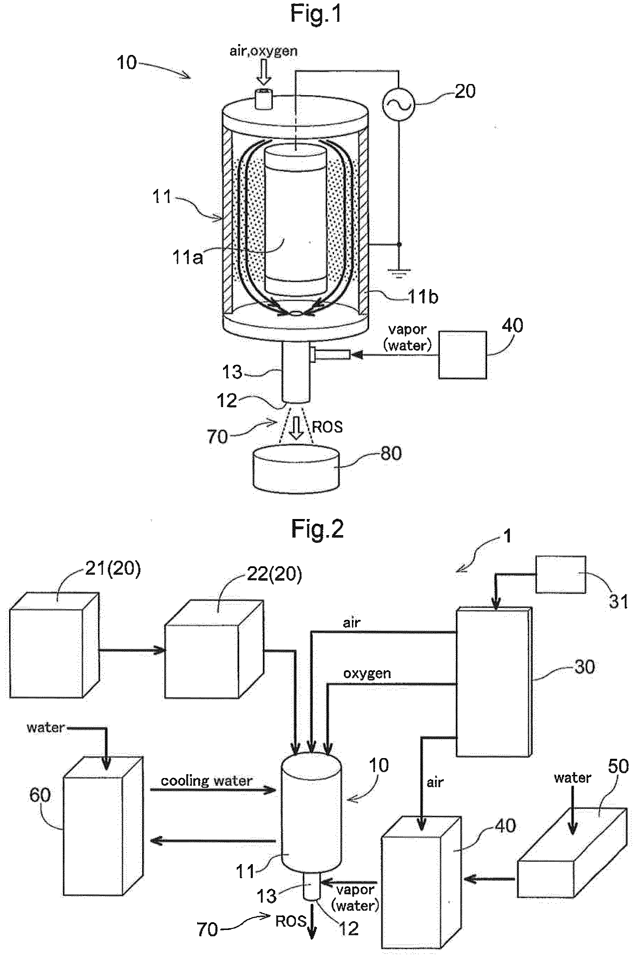

Fig. 1 shows a production mechanism for reactive oxygen in the sterilizing device 1. The sterilizing device 1 includes a nozzle 10 configured to produce reactive oxygen and discharge (irradiate) sterilizing agent 70 containing the reactive oxygen to the caps 80. This nozzle 10 includes a plasma producing unit 11 for producing plasma, an outlet 12 for discharging the sterilizing agent 70 containing the plasma and the reactive oxygen, and a relaying section 13 between the plasma producing unit 11 and the outlet 12.

This nozzle 10 produces so-called atmospheric pressure plasma within the device. By using the atmospheric pressure plasma, e.g. a vacuum vessel required for low pressure plasma can be omitted, so that the device cost can be reduced. Further, as the production process is continuous, the work efficiency is high. Moreover, since the production is possible even at a low temperature, there is obtained a further advantage of not needing to expose the process-subject article to a high temperature. Next, the production of such high atmospheric pressure plasma (to be referred to simply as "plasma" hereinafter) and production of reactive oxygen using plasma will be explained.

The plasma producing unit 11 has a well-known construction including an internal electrode 11a and an external electrode 11b. In the plasma generating unit 11, by an AC power source 20, a high voltage (e.g. effective voltage of 20 kV at frequency of 14 kHz) is applied between the internal electrode 11a and the external electrode 11b, whereby an electric field is generated within the plasma producing unit 11. And, into the plasma producing unit 11, gas together with air is fed to pass this gas through the generated electric field, thus producing plasma. The produced plasma is then sent to the relaying section 13. In the instant embodiment, into the plasma producing unit 11, oxygen (O₂) is supplied as an example of "gas", so that ozone-containing plasma is produced as "plasma" inside the plasma producing unit 11. More particularly, by the plasma production process, oxygen radicals and ozone (O₃) are generated, which are then sent to the relaying section 13.

The relaying section 13 is connected to an evaporator (an example of steam feeding unit, which will be detailed later) 40 and steam is also sent to the relaying section 13. Within the relaying section 13, the plasma (oxygen radical and ozone) sent from the plasma producing unit 11 and the steam (vapor) sent from the evaporator 40 react with each other, thus producing reactive oxygen. In the instant embodiment, an arrangement is provided such that as the oxygen plasma (oxygen radical and ozone) and the steam are caused to react with each other, there is mainly produced hydroxy radical (·OH) which has a particularly high reactivity among reactive oxygen species.

More specifically, with the reaction between steam and plasma, hydrogen radical (·H) and hydroxy radical are produced as shown by Formula (1) below.

H₂O→ ·H+ ·OH...... (1)

Also, the hydrogen radical reacts with the ozone to produce hydroxy radical and oxygen (O₂) as shown by Formula (2) below.

H + O₃ → ·OH + O₂ ...... (2)

Formula (1) and Formula (2) can be combined into Formula (3) below.

H₂O + O₃ → 2 ·OH + O₂...... (3)

Namely, with the reaction between oxygen plasma and steam, the reaction according to Formula (3) is caused to take place, whereby hydroxy radical (· OH) can be produced in an efficient manner. As a result, the reactive oxygen thus produced contains mainly hydroxy radical having such particularly high reactivity. And, as it contains mainly such hydroxy radical having particularly high reactivity, a high sterilization effect can be achieved. And, such produced reactive oxygen, steam and unreacted plasma will be discharged together as the sterilizing agent 70 through the outlet 12 to the caps 80, whereby the caps 80 are sterilized.

Next, a device configuration shown in Fig. 2 of the sterilizing device 1 for effecting sterilization using reactive oxygen will be explained. The sterilizing device 1 includes, in addition to the nozzle 10, a generator 21 and a transformer 22 which together constitute the AC power source 20, a gas supplying unit 30 for feeding various kinds of gas to the nozzle 10 and to the evaporator 40, the evaporator 40 for feeding steam to the relaying section 13, a pump 50 for feeding water to the evaporator 40, and a chiller 60 for feeding cold water to the nozzle 10.

The generator 21 generates an alternating current. For instance, in this embodiment, there is employed one having a frequency of 14 kHz, an effective voltage of 300V and an effective current of 11A. Then, the alternating current supplied by the generator 21 is boosted from 300 V to 20 kV by the transformer 22. With this, a high voltage of 20 kV is applied between the internal electrode 11a and the external electrode 11b in the plasma producing unit 11.

The gas supplying unit 30 supplies oxygen (O₂) together with air to the nozzle 10 and also supplies air for feeding the steam produced by the generator 40 to the relaying section 13. The gas supplying unit 30 includes a control panel 31. By operating this control panel 31, a supplying amount of the various gases to the respective components can be controlled. In this embodiment, by operating the control panel 31, for instance, air at 6 L/min and oxygen at 3L/min can be respectively sent to the nozzle 10 and air at 3L/min can be sent to the evaporator 40.

The evaporator 40 is arranged such that an electric heating wire incorporated therein (not shown) is heated to 300°C, thus heating water supplied from the pump 50 with this heated wire to generate steam and as this steam is mixed with the air supplied from the gas supplying unit 30, the steam together with the air will be supplied to the relaying section 13. Incidentally, in this embodiment, the pump 50 is configured to supply water at 1.2 ml/min to the evaporator 40.

The chiller 60 is configured to cool the nozzle 10 by supplying cold water thereto, which nozzle 10 has been heated with the application of the high voltage.

In operation with the sterilizing device 1 configured as described above, oxygen supplied together with air from the gas supplying unit 30 to the nozzle 10 is converted into plasma at the plasma producing unit 11 and at the relaying section 13, resultantly produced oxygen plasma is reacted with the steam supplied from the evaporator 40 together with air, thus continuously producing reactive oxygen containing hydroxy radical as the main component thereof. Then, the reactive oxygen, steam and unreacted plasma produced continuously at the relaying section 13 will be discharged continuously via the outlet 12, thus enabling continuous treatment of the caps 80. In this embodiment, for instance, sterilizing agent 70 containing plasma and the reactive oxygen will be discharged via the outlet at a flow rate of 50000 mm/sec at a temperature ranging from 50 to 80 °C.

Effecting sterilization with using reactive oxygen provides the following advantages. If a sterilizing agent containing hydrogen peroxide is used, it is necessary to effect a cleaning operation thereafter so that the sterilizing agent will not remain on the sterilization-subject article. However, elimination of all sterilizing agent by cleaning is difficult, so there is a risk of some sterilizing agent remaining on the sterilization-subject article. Also, since spraying of the sterilizing agent and the cleaning subsequent thereto are effected at a high temperature, if the sterilization-subject article is made of a material such as a resin which suffers thermal contraction, the sterilization process will be subjected to various limitations in order to prevent excessive thermal contraction or deformation. On the other hand, reactive oxygen dies out over time, so it will not remain. Moreover, sterilization using reactive oxygen does not require application of such heat which may cause thermal contraction of the sterilization-subject article. Therefore, it is possible to avoid residual of the sterilizing agent on the sterilization-subject article and occurrence of excessive thermal contraction/deformation of the sterilization-subject article.

Next, the configuration of the sterilizing system 100 relating to this embodiment will be explained. Referring firstly to its principal components, the sterilizing system 100 relating to this embodiment includes a conveying unit 110 for conveying the caps 80, and the sterilizing devices 1 configured to discharge the sterilizing agent 70 onto the conveyed caps 80 over the span of a predetermined section D. The system 100 further includes a sterilizing mechanism configured to be able to sterilize at least an open face (corresponding to "one side") 81 and a top face (corresponding to "the other side") 82 of each cap 80 in the span of the predetermined section D. Next, this sterilizing system 100 will be explained more particularly.

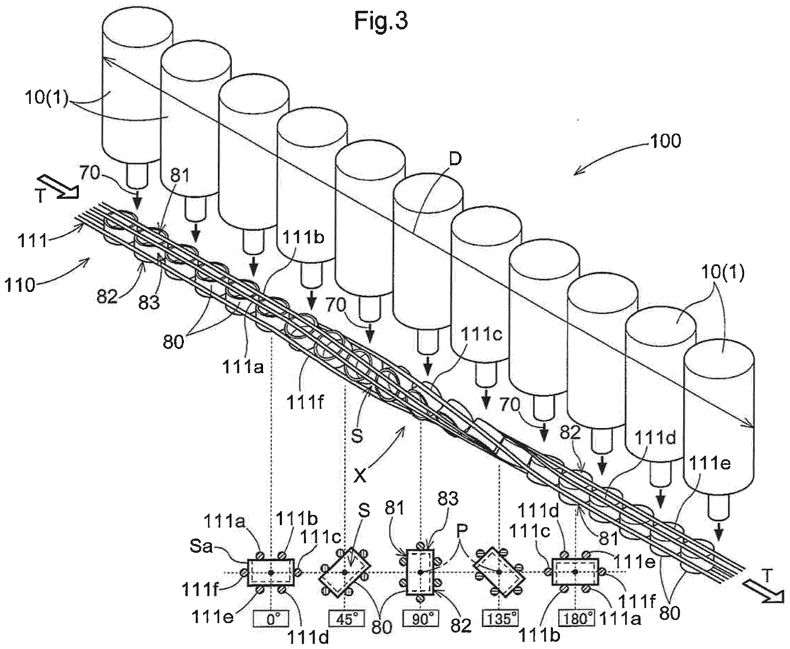

As shown in Fig. 3, in this sterilizing system 100, a plurality of sterilizing devices 1 are disposed in the span of the predetermined section D along the conveying unit 110 (in Fig. 3, only the nozzles 10 of the sterilizing devices 1 are shown for the sake of easier understanding). With this arrangement the sterilizing agent 70 can be discharged onto the caps 80 being conveyed over the span of the predetermined section D. In each sterilizing device 1, the respective outlet 13 thereof is oriented downwards. So, the sterilizing agent 70 is discharged downwards. This is because of the following reason. Namely, since a high voltage is applied in the plasma producing unit 11 inside the sterilizing device 1, it is extremely dangerous if water or the like may be present therein. Thus, the arrangement is provided for preventing mixing intrusion thereof into the device. To this end, although certain change in its orientation may be permissible, it is still safe to effect the sterilization by orienting the outlet for discharging the sterilizing agent downwards.

The conveying unit 110 is comprised of a plurality (six in total in this embodiment) of rod-like members 111 which respectively extend along a conveying direction T along the span of the predetermined section D. As shown in Fig. 3, the six rod-like members 111 (111a-111f) are disposed as being spaced apart from each other in a annular form around the shapes of the caps 80 over the span of the predetermined section D. Referring more particular to the respective rod-like members 111, two of them (111a and 111b) are disposed on the open face 81 side of the cap 80, a further two of them (111d and 111e) are disposed on the top face 82 side the cap 80, and of the other remaining two, one is disposed on the left side and the other one is disposed on the right side for the lateral face thereof. In operation, as each cap 80 is being guided by the six rod-like members 111, the cap 80 is conveyed within an inner space S delimited by these rod-like members 111 altogether. Namely, the inner space S constitutes a "conveying path" for the cap 80.

The sterilizing system 100 includes, in the span of the predetermined section D, an inverting mechanism X configured to at least invert the cap 80 in the span of this predetermined section D, as a sterilizing mechanism capable of sterilizing both the open face 81 and the top face 82 of the cap 80. Referring more particularly to this sterilizing mechanism, as shown in Fig. 3, by causing the respective rod-like members 111 together to effect semi-rotation helically about a centerline (the straight line extending through the center of the inner space S) P as a rotational axis, the inner space S as the conveying path of the caps is inverted. With this arrangement, in association with their advance in the conveying direction, the six rod-like members will have their positions changed one after another counterclockwise altogether about the center axis P of the inner space S as seen from the side of the advancing direction. And, in association with this, the inner space too effects a half rotation (180 degrees) eventually, whereby the position of each rod-like member 111 moves to the position in symmetry relative to the center axis P and its cross sectional shape Sa is inverted from its initial position.

Therefore, as the cap 80 is advanced through the inner space S of the six rod-like members 111, and the inner space S is rotated counterclockwise in association with this advance, the cap 80 too is rotated counterclockwise and eventually inverted. Namely, at the initial state in the predetermined section D, the cap 80 has the posture of its open face 81 oriented upwards (see state 0° in Fig. 3). So, its open face 81 side (that is, the inner face of the cap 80) is sterilized. Further, in the course of this, the sterilizing agent 70 is dispersed about the cap 80, so by such dispersed sterilizing agent 70, the lateral face of the cap 83 too will be sterilized. Thereafter, as its advance in the predetermined section D, the cap 80 will be rotated progressively (see states of 45° to 135° in Fig. 3), so the sterilizing agent 70 will be discharged to its lateral face 83 also. And, eventually, the cap 80 will be inverted to orient its top face 82 upwards (see state of 180°in Fig. 3). Thereafter, this top face 82 side will be sterilized. As a result, the entire cap 80 will be sterilized in an efficient manner. In this, since the rod-like members 111 are spaced apart from each other, the sterilizing agent 70 is applied through the gaps formed between these rod-like members 111, so the rod-like members 111 present no obstacle in the sterilization process.

Incidentally, as a method of propelling the cap 80 among the rod-like members 111, there can be cited a method of uniformly inclining the respective rod-like members 111 (conveying path 110), so that the caps 80 can be propelled with utilization of their own weights or a method of propelling the caps 80 by feeding compressed air to them from behind thereof.

As described above, according to the sterilizing system 100 relating to the present embodiment, the cap 80 can be sterilized on its open face 81 side and its top face 82 side opposite thereto, so that the cap 80 can be sterilized from both sides. Further, respecting its lateral face 83, in the course of the inversion, the sterilizing agent 70 can be applied to one lateral side, thus the lateral face 83 can be sterilized with the dispersed sterilizing agent 70. With this, the entire cap 80 can be sterilized in an efficient manner. Further, the cap 80 can be sterilized from both sides with only the inversion, without needing to provide some special additional mechanism to the sterilizing device 1 side. Thus, the system can be suitably applied to a case wherein the sterilizing agent 70 is discharged from one direction only, which is the case with a sterilizing device 1 having restriction in the discharging direction of the outlet for discharging the sterilizing agent 70, from the viewpoint of safety problem.

### Other Embodiments

Lastly, other embodiments of the sterilizing system relating to the present invention will be explained. Incidentally, the arrangements/configurations to be disclosed in the following embodiments can be used in combination with the arrangements/configurations disclosed in the other embodiment's) in any desired manner, as long as no contraction occurs as a result of such combination.
(1) In the foregoing embodiment, there was disclosed an exemplary arrangement in which by arranging a plurality of sterilizing devices 1 along the conveying unit 110 in the span of the predetermined section D, it is made possible to discharge the sterilizing agent 70 onto the conveyed caps 80 over the span of the predetermined section D. However, embodiments of the present invention are not limited thereto. For instance, only one sterilizing device 1 can be installed and discharging of the sterilizing agent 70 can be effected while following the caps 80 which are being conveyed, thus making discharging of the sterilizing agent 70 to the caps 80 over the span of the predetermined section D possible.
(2) In the foregoing embodiment, there was disclosed an exemplary arrangement in which the conveying unit 110 is comprised of a plurality of rod-like members 111 and by causing the respective rod-like members 111 to effect a half rotation altogether, the conveyed caps 80 are inverted in the span of the predetermined section D. However, embodiments of the present invention are not limited thereto. For instance, the inversion of the cap 80 can be effected with simultaneously gripping of this cap 80 by an arm or the like. Further, the embodiment is not limited to half rotation, but can be one full rotation or one and a half rotation, thus, there is no particular limitation as long as it is rotated through 180 degrees plus.
(3) In the foregoing embodiment, there was disclosed an exemplary arrangement in which the outlets 12 of the sterilizing devices 1 are oriented downwards. However, embodiments of the present invention are not limited thereto. For instance, in the span of the predetermined section D, the discharging angle of the sterilizing agent 70 of the sterilizing device 1 to the conveying unit 110 may be varied. With this, the sterilizing agent 70 can be discharged in a variety of angles to the caps 80. And, if this is used in combination with the inverting mechanism X, discharging of the sterilizing agent to the sterilization-subject article from a wider angle direction is made possible. For example, depending on such combination, it will be made possible also to discharge the sterilizing agent 70 onto the entire surface of the cap 80. Incidentally, due to the safety requirement, the plasma sterilizing device 1 was set with its outlet 12 being oriented downwards. However, since certain variation of its orientation is permissible (inclining it by 45 degrees, for instance), variation in the discharging angle of the sterilizing agent 70 to the conveying unit 110 is possible. As some examples of such variation of the discharging angle, it is possible e.g. to change the orientation of the outlet 12 in the left-right direction relative to the conveying direction, thereby to vary the discharging direction of the sterilizing agent 70 so that the sterilizing agent may be discharged to the cap 80 from the right side or left side relative to the conveying direction T or to inline the outlet 12 in the direction parallel with the conveying direction T, thus varying the discharging angle of the sterilizing agent 70 to the conveying direction 110, so that the sterilizing agent may be discharged to the cap 80 from the front side or rear side relative to the conveying direction T. Incidentally, inclination can be provided in a plurality of stages by e.g. inclining the relaying section 13 in two stages. When a plurality of sterilizing devices 1 are provided, it is possible to arrange such that the discharging angles of the respective sterilizing devices 1 may be variably adjusted. Further, the discharging angles of the respective sterilizing devices 1 may be fixed at discharging angles different from each other, so that the sterilizing agent may be discharged from a variety of directions in the course of conveying of the cap 80. When the cap 80 is caused to follow displacement/movement of a single sterilizing device, arrangement may be provided for varying its discharging angle in the course of such follow-up movement.
(4) In the foregoing embodiment, there was disclosed an exemplary arrangement in which as the sterilizing device, there was employed a plasma sterilizing device 1 configured to effect sterilization with using reactive oxygen, and a cap 80 was used as the sterilization-subject article. However, embodiments of the present invention are not limited thereto. For instance, as the sterilizing device, it is possible to employ a device configured to effect sterilization by a different technique, such as a device configured to effect sterilization with hydrogen peroxide. Moreover, the kind of the sterilization-subject article can vary as needed.

Respecting the other arrangements/configurations too, the embodiments disclosed in this detailed description are only exemplary, and the scope of the present invention is not limited thereto. One skilled in the art will readily understand that other modifications will be made possible as needed or desired without departing from the essence of the present invention. Therefore, it is understood that such other variations and modified embodiments made without departing from the inventive essence are also intended be included in the scope of the present invention.

### INDUSTRIAL APPLICABILITY

The present invention is applicable to a sterilizing system for sterilizing a sterilization-subject such as a cap.

### Description of Reference Marks/Numerals

1: plasma sterilizing device (sterilizing device)
12: outlet
D: predetermined section
70: sterilizing agent
80: cap (sterilization-subject article)
81: open face (one side)
82: top face (the other side)
110: conveying unit
111: rod-like member
S: inner space
X: inverting mechanism (sterilizing mechanism)

## Claims

1. A sterilizing system comprising:
a conveying unit for conveying a sterilization-subject article;
a sterilizing device configured to discharge sterilizing agent onto the conveyed sterilization-subject article over a span of a predetermined section; and
a sterilizing mechanism provided in the span of the predetermined section and configured to be able to sterilize at least one side and the other side of the sterilization-subject article.

2. A sterilizing system of claim 1 wherein the sterilizing mechanism comprises an inverting mechanism configured to at least invert the sterilization-subject article in the span of the predetermined section.

3. A sterilizing system of claim 2 wherein:
the conveying unit comprises, at least in the span of the predetermined section, a plurality of rod-like members each extending along a conveying direction;
the plurality of rod-like members are spaced from each other and are arranged in a annular configuration along a shape of the sterilization-subject article;
the sterilization-subject article is conveyed within an inner space delimited by the plurality of rod-like members while being guided by these rod-like members; and
the inverting mechanism is configured to helically invert the respective rod-like members all together in the predetermined section for at least a half rotation.

4. A sterilizing system of claim 2 or 3 wherein:
the sterilizing device comprises a plasma sterilizing device configured to generate plasma and to sterilize the sterilization-subject article with the obtained plasma;
the plasma sterilizing device receives a supply of steam and the plasma sterilizing device is configured to discharge generated plasma and reactive oxygen, produced through reaction between the supplied steam and the generated plasma, as the sterilizing agent; and
an outlet of the plasma sterilizing device for discharging the sterilizing agent is oriented perpendicularly downwards.

5. A sterilizing system of any one of claims 1-4, wherein the sterilizing mechanism is configured to vary a discharging angle of the sterilizing agent relative to the conveying unit, in the span of the predetermined section.
